# EUROPÄISCHE PATENTANMELDUNG

(11) **EP 3 819 259 A1**
(43) Veröffentlichungstag der Anmeldung: **12.05.2021**
(21) Anmeldenummer: 19207406.0
(22) Anmeldetag: 06.11.2019
(51) Int. Cl.: C01B 3/12, C01B 3/50, C01B 7/04, C01B 32/80, C07C 263/10, C08G 18/10, C25B 1/04, C09D 175/04

(54) **VERFAHREN ZUR ISOCYANAT- UND POLYURETHAN-HERSTELLUNG MIT VERBESSERTER NACHHALTIGKEIT**

(71) Anmelder: Covestro Deutschland AG, 51373 Leverkusen (DE)
(72) Erfinder: Die Erfindernennung liegt noch nicht vor
(74) Vertreter: Levpat

(57) **Zusammenfassung**

Es wird ein Verfahren zur Herstellung von Isocyanaten und Polyurethanen beschrieben durch Synthese (1) von Phosgen (20) aus Kohlenmonoxid (21) und Chlor (22), Umsetzung (2) von Phosgen (20) mit Diaminen (23) zu Diisocyanaten (24) und Chlorwasserstoff (25), Umsetzung (3) der Diisocyanate (24) mit Polyetherpolyol (35a) und/oder Polyesterpolyol (35b) zu Polyurethanen (37), Bereitstellung eines Kohlendioxid Gasstroms (31) und Reinigung (4) des Kohlendioxid Gasstroms (31) von Nebenbestandteilen, in dem das Kohlendioxid mittels einer RWGS- Reaktion (6) zu Kohlenmonoxid (21) und Wasserstoff (29) umgesetzt wird, welche als Rohstoffe für die Polyurethan-Herstellung eingesetzt werden.

## Beschreibung

Die Erfindung betrifft ein Verfahren zur Herstellung von Isocyanaten und Polyurethanen durch Synthese von Phosgen aus Kohlenmonoxid und Chlor, Umsetzung von Phosgen mit Diaminen zu Diisocyanaten und Chlorwasserstoff, Umsetzung der Diisocyanate mit Polyethern und/oder Polyestern zu Polyurethanen, Bereitstellung eines Kohlendioxid Gasstroms und Reinigung des Kohlendioxid Gasstroms von Nebenbestandteilen und anschließende Umsetzung des Kohlendioxids zur Herstellung von Kohlenmonoxid, welches in der Phosgensynthese eingesetzt wird.

Die Erfindung betrifft weiter die Verwertung von Polyurethan-enthaltenden Abfall-Materialien (nachfolgend auch als "Polyurethan-Material Abfall" bezeichnet) zur Herstellung von chemikalischen Rohstoffen für die Herstellung von Isocyanaten und anschließend Polyurethanen, bei dem ausgehend von Polyurethan-Material Abfall z. B. durch Pyrolyse, Kohlendioxid und Kohlenwasserstoffe und gegebenenfalls Kohlenmonoxid und Wasserstoff erzeugt werden, das Kohlendioxid durch Umsetzung mit Wasserstoff zu Kohlenmonoxid in einer sogenannten Reversen Wassergas Shift Reaktion (nachfolgend RWGS Reaktion genannt) erfolgt und das erhaltene Kohlenmonoxid über Phosgen zu Isocyanat umgesetzt wird und das Isocyanat zu neuem Polyurethan-Material weiterverarbeitet werden kann.

Die Erfindung betrifft besonders ein Verfahren zur emissionsarmen Herstellung von Isocyanaten unter Einsatz einer RWGS Reaktion und der Bereitstellung von Wasserstoff aus einer Wasserelektrolyse oder aus einer Elektrolyse zur Herstellung von Chlor, sowie der Nutzung des Sauerstoffs aus der Wasserelektrolyse zur Verbrennung von Polyurethan enthaltenen Materialien zu Kohlendioxid und ggf. Verbrennung von Pyrolyserückständen erhalten aus Polyurethan enthaltenen Materialien und Nutzung des jeweilig erhaltenen Kohlendioxids als Rohstoff für die RWGS Reaktion.

Das, bevorzugt aus der Verwertung des Polyurethan-Material Abfalls hergestellte, Kohlenmonoxid wird mit Chlor zu Phosgen und dieses mit Aminen zu Isocyanaten umgesetzt. Aus den Isocyanaten können durch Umsetzung mit Polyetherpolyol bzw. Polyesterpolyol erneut Polyurethan-Materialien hergestellt werden. Somit ist ein Teil der Wertschöpfungskette geschlossen. Bei Einsatz von CO₂ und elektrischem Strom aus regenerativen Energiequellen für die Wasserelektrolyse kann Polyurethan Material mit weiter verbesserter Nachhaltigkeit hergestellt werden. Der Anteil von fossilem Kohlenstoff im Polyurethan soll deutlich vermindert werden.

Weiter kann durch die Wasserelektrolyse zusätzlich benötigter Wasserstoff zur Hydrierung der NitroVerbindungen zu Aminen hergestellt werden, die mit dem Phosgen zu Isocyanaten umgewandelt werden können. Bei der Wasserelektrolyse wird das Koppelprodukt Sauerstoff an der Anode erzeugt. Dieser Sauerstoff kann für die Verbrennung der Polyurethan-enthaltenden Abfall-Materialien und des Pyrolyserückstands eingesetzt werden, wodurch bei der Verbrennung ein hochkonzentrierter CO₂ Abgasstrom erhalten wird und die CO₂ Rückgewinnung hierdurch deutlich wirtschaftlicher wird, als bei der Verbrennung des Polyurethan-Material enthaltenen Abfalls mit Luft. Es kann jedoch auch CO₂ aus alternativen Quellen wie z.B. der Verbrennung anderer Abfälle eingesetzt werden. Das CO₂ wird gereinigt und der RWGS Reaktion zugeführt.

Polyurethane, nachfolgend auch abgekürzt PU genannt, sind Kunststoffe, die aus der Polyadditionsreaktion von mindestens zwei Hydroxylgruppen enthaltenden Polyolen mit Polyisocyanaten entstehen. Der Einsatz von Diolen und Diisocyanaten führt zu linearen Polyurethanen. Vernetzte Polyurethane können durch Umsetzung von Triisocyanat-Diisocyanat-Gemischen mit Triol-Diol-Gemischen hergestellt werden. Die Eigenschaften von PU können in einem weiten Rahmen variiert werden. Je nach Vernetzungsgrad und/oder eingesetzter Isocyanat- oder OH-Komponente erhält man Duroplaste, Thermoplaste oder Elastomere. Polyurethane werden jedoch auch als Formmassen zum Formpressen, als Gießharze (Isocyanat-Harze), als (textile) elastische Faserstoffe, Polyurethanlacke und als Polyurethanklebstoffe verwendet. Aus Polyurethan lassen sich auch sehr einfach Schaumstoffe herstellen.

Weiche PU-Schaumstoffe werden für sehr viele Zwecke verwendet, vor allem als Polstermaterial z. B. für Möbel und Autositze, als Matratzenschaum, als Teppichrückenmaterial, zur Textilkaschierung, als Reinigungsschwamm oder als Filtermaterial.

PU-Hartschäume werden vor allem zur Wärmedämmung z. B. in Gebäuden, Kühlgeräten, Wärme- und Kältespeichern sowie einigen Rohrsystemen (Kunststoffmantelverbundrohr, flexible Verbundrohre) eingesetzt.

Weitere, relativ neue Anwendungsgebiete für PU-Schäume gibt es im Fahrzeugbau wie Lenkrad, Armauflage, Softbeschichtung von Handgriffen, Innenraumverkleidung, Armaturenbrett, Schalldämmung, Klapperschutz, Abdichtungen, Transparentbeschichtung von Holzdekoren.

Nach dem Ablauf der Nutzungsphase der PU-Material enthaltenen Produkte werden diese üblicherweise entsorgt, d.h. auf Mülldeponien gelagert oder in Müllverbrennungsanlagen verbrannt. Eine stoffliche Nutzung konnte bisher noch nicht wirtschaftlich erfolgreich durchgeführt werden, d.h. dass es bisher nicht gelang, aus den PU-Materialien die eingesetzten Polyole bzw. Polyisocyanate in wirtschaftlicher Ausbeute wieder zu gewinnen.

Ein Ansatz zum stofflichen Recycling von PU ist die Glykolyse, bei der die Urethangruppe mit Glykol zu Carbamat und einem Polyol umgesetzt wird.

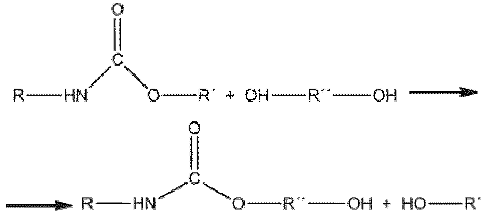

Weiterhin kann die Urethangruppe mit einem Amin zu Urea und einem Polyol umgesetzt werden.

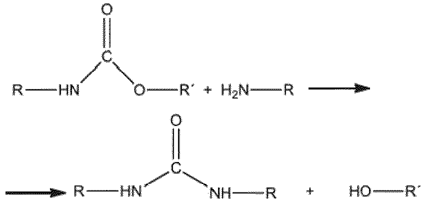

Aufgabe der vorliegenden Erfindung war es, ein nachhaltigeres Verfahren für die Isocyanatherstellung und letztlich auch für die Polyurethan-Herstellung unter Einbeziehung von Recyclingprozessen und Schließung der Wertschöpfungsketten zu finden. Bisher werden wesentliche Komponenten zur Polyurethanherstellung wie das Kohlenmonoxid, Wasserstoff oder der Strom zum Betrieb der Elektrolysen wie der Wasserelektrolyse und der Chlor-Alkali-Elektrolyse aus fossilen Energieträgern hergestellt. So wird konventionell Kohlenmonoxid und Wasserstoff aus Erdgas bzw. aus Kohle mittels Reformingprozessen, und Chlor aus der Elektrolyse mit Strom, der unter Einsatz von fossilen Brennstoffen wie Öl, Kohle oder Erdgas hergestellt wurde, gewonnen.

Unter "Nachhaltigkeit" eines Verfahrens versteht der Fachmann in Anwendung der durch die UN geprägte Nachhatigkeits-Definition ("sustainable development") gemäß Brundtlandbericht der "Weltkommission für Umwelt und Entwicklung", dass durch die Ausführung des Verfahrens in der Gegenwart ein möglichst geringer bis gar kein Beitrag geleistet wird, dass künftige Generationen der Menschheit ihre eigenen Bedürfnisse nicht mehr befriedigen können, insbesondere Bedürfnisse mit Blick auf die Nutzung von Ressourcen wie z.B. fossiler Rohstoffe und insbesondere mit Blick auf die Schonung des Lebensraumes, wie z.B. den Schutz der Erdatmosphäre. Die Erfindung hat somit zur Aufgabe, die Produktion von Isocyanat und Polyurethan nachhaltiger als die aus dem Stand der Technik bekannten Produktionsmethoden zu gestalten. Der Beitrag der Produktion von Isocyanat und Polyurethan zu einer sinkenden Befriedigung der Bedürfnisse zukünftiger Generationen soll vermindert bis vermieden werden.

Eine Aufgabe der Erfindung ist es also, den Einsatz von fossilen Rohstoffen als Edukt für die Isocyanatproduktion und gegebenenfalls auch den Einsatz von fossilen Rohstoffen zur Bereitstellung von Energie für die Isocyanatproduktion zu vermindern. Insbesondere durch letztere Aufgabenstellung soll die Kohlendioxid-Bilanz (Carbon Footprint) der PU-Produktion zum Schutz der Erdatmosphäre weiter verbessert werden.

Gegenstand der Erfindung ist ein Verfahren zur Herstellung von Isocyanaten und Polyurethanen durch
Synthese von Phosgen aus Kohlenmonoxid und Chlor,
Umsetzung von Phosgen mit Diaminen zu Diisocyanaten und Chlorwasserstoff,
Umsetzung der Diisocyanate mit Polyetherpolyol und/oder Polyesterpolyol zu Polyurethanen,
Bereitstellung eines CO₂ Gasstroms,
Reinigung des CO₂ Gasstroms von Nebenbestandteilen, insbesondere von Stickoxiden, Schwefelverbindungen, Staub, Wasser, Sauerstoff und HCl wahlweise mittels Adsorption, Gaswäsche oder katalytischer Behandlung unter Erhalt eines gereinigten Kohlendioxids,
Elektrolyse von Wasser zu Wasserstoff und Sauerstoff,
Bereitstellung des Wasserstoffstroms und Einspeisung zusammen mit dem gereinigten CO₂ Gasstrom in eine RWGS Reaktionszone und Umsetzung der Edukte nach dem Prinzip der RWGS Reaktion zu einem Produktgasgemisch aus Wasserdampf, CO und gegebenenfalls Nebenprodukten, insbesondere niedere Kohlenwasserstoffe, insbesondere bevorzugt Methan,
Abtrennung des Wassers des Wasserdampfes aus dem Produktgasgemisch und Rückführung des Wassers in die Wasserelektrolyse,
Abtrennung von nicht umgesetztem Kohlendioxid aus dem aus der Abtrennung erhaltenen Gasgemischs der RWGS Reaktion, insbesondere mittels Aminwäsche, und Rückführung des nicht umgesetzten Kohlendioxids in die RWGS Reaktion,
Abtrennung des in der RWGS Reaktion nicht umgesetzten Wasserstoffs aus dem nach der Abtrennung erhaltenen Gasgemisch von Kohlenmonoxid und Wasserstoff, insbesondere unter Verwendung einer Coldbox, und wahlweise Rückführung des Wasserstoffs in die RWGS Reaktion oder Einspeisung des Wasserstoffs in die Hydrierung von Di-nitroverbindungen zur Herstellung von Diaminen als Rohstoff für das Diisocyanat,
Einspeisung des verbliebenen Kohlenmonoxids aus der Abtrennung in die Phosgensynthese,
Einspeisung des Wasserstoffs aus der Wasserelektrolyse, gegebenenfalls zusammen mit nicht umgesetztem Wasserstoff aus der RWGS Reaktion in die Hydrierung von Nitroverbindungen zur Herstellung von Diaminen,
Abtrennung und Reinigung des in der Isocyanat-Herstellung gebildeten Chlorwasserstoffs und anschließende oxidative Umsetzung des Chlorwasserstoffs als Umsetzung in einer thermokatalytischen Gasphasenoxidation mit Sauerstoff zu Chlor und Wasser und/oder als elektrochemische Oxidation des Chlorwasserstoffs zu Chlor und/oder als elektrochemische Oxidation des Chlorwasserstoffs zu Chlor und Wasserstoff mittels HCl Diaphragmaelektrolyse,
Zuführung des zuvor gebildeten Chlors in die Phosgensynthese gegebenenfalls unter Zuspeisung von frischem Chlor aus einer Chloralkali-Elektrolyse.

Unter "niederen Kohlenwasserstoffen" werden erfindungsgemäß Kohlenwasserstoffe mit 1 bis 8 Kohlenstoffatomen verstanden.

Unter "Aminwäsche" des Produktgases der RWGS Reaktion wird hier insbesondere die grundsätzlich bekannte Wäsche des Gasgemisches nach dem Prinzip der Chemisorption mit Aminen wie Monoethanolamin (MEA) Diethanolamin (DEA), Methyldiethanolamin (MDEA) oder Diglycolamin (DGA) verstanden, welche schon bei relativ niedrigem Druck in einer Absorptionskolonne eine hohe Reinheit des gereinigten Gasgemisches erreicht.

Unter "regenerativer Energie" versteht der Fachmann Energie aus einer Energiequelle, die sich nicht erschöpft, wie z.B. Windenergie, Wasserenergie oder Sonnenenergie.

Ein bevorzugte Ausführungsform des erfindungsgemäßenVerfahrens ist dadurch gekennzeichnet, dass für die RWGS-Synthese Kohlendioxid verwendet wird, das aus der Verwertung von Polyurethan-Material Abfall durch Verbrennung und/oder durch Pyrolyse entsteht. Dabei ist es wiederum bevorzugt, wenn bei der Verbrennung Sauerstoffgas genutzt wird, das aus der Wasserelektrolyse erhalten wird.

Ganz besonders bevorzugt ist ein Verfahren, bei dem für die RWGS-Synthese Kohlendioxid verwendet wird, das aus der Verwertung von Polyurethan-Material Abfall durch Verbrennung in Gegenwart von Gas mit einem Sauerstoffgas-Gehalt (O₂) entsteht, wobei besagtes Gas einen Sauerstoffgas-Gehalt (O₂) von mindestens 30 Vol.%, bevorzugt von mindestens 50 Vol.%, besonders bevorzugt von mindestens 95 Vol.%, ganz besonders bevorzugt von mindestens 99 Vol.%, am bevorzugtesten von mindestens 99,5 Vol.%, aufweist.
Das dabei zur Verbrennung eingesetzte Sauerstoffgas kann wiederum bevorzugt aus der Wasserelektrolyse erhalten werden.

Die Verwertung des Polyurethan-Material Abfalls erfolgt beispielsweise durch Pyrolyse des besagten Polyurethan-Material Abfalles bei erhöhter Temperatur, gegebenenfalls in Gegenwart von Katalysator, unter Erhalt von Kohlendioxid, gegebenenfalls Kohlenmonoxid, gegebenenfalls Wasserstoff, gegebenenfalls einem Gemisch von aliphatischen und aromatischen niedermolekularen Kohlenwasserstoffen und stickstoffhaltigen Kohlenwasserstoffen und gegebenenfalls einem Rückstand von höher molekularen Kohlenwasserstoffen. Das in der Pyrolyse erhaltene Gemisch wird bevorzugt anschließend einer Raffination unter Erhalt eines Gasgemisches von Kohlendioxid, Kohlenmonoxid, Wasserstoffgas und weiteren bei Normalbedingungen gasförmigen niedermolekularen Kohlenwasserstoffverbindungen.

Die Verbrennung des in der Pyrolyse erhaltenen Rückstands und gegebenenfalls von weiterem Polyurethan-Material Abfall kann insbesondere mit sauerstoffhaltigem Gas, insbesondere mit reinem Sauerstoff, unter Erhalt von Kohlendioxid enthaltendem Gas erfolgen.

In einer bevorzugten Ausführung des neuen Verfahrens wird für die RWGS-Synthese Kohlendioxid verwendet, das aus der Verbrennung von Polyurethan-Material Abfall unter Verwendung von Sauerstoff entsteht, der aus der Wasserelektrolyse erhalten wird.

In einer weiteren bevorzugten Ausführung des neuen Verfahrens wird die Wasserelektrolyse und/oder die elektrochemischen Oxidation unter Verwendung von aus regenerativer Energie erzeugtem elektrischen Strom durchgeführt, insbesondere von elektrischem Strom wahlweise erhalten durch Nutzung von Windkraft, Sonnenenergie oder Wasserkraft.

In einer anderen bevorzugten Ausführungsform des neuen Verfahrens werden die Wasserelektrolyse und/oder die elektrochemische Oxidation unter Verwendung von elektrischem Strom aus rückgekoppelter Energie durchgeführt, die bei der Verbrennung von gebrauchtem Polyurethan-Material und/oder der Durchführung der RWGS Reaktion erhalten wird.

Eine weitere alternative Ausführungsform des neuen Verfahrens ist dadurch gekennzeichnet, dass die RWGS Reaktion mittels aus regenerativer Ernergie erzeugtem elektrischen Stroms durchgeführt wird, insbesondere elektrischem Strom wahlweise erhalten durch Nutzung von Windkraft, Sonnenenergie oder Wasserkraft.

In einer weiteren alternativen Ausführung des neuen Verfahrens wird die Heizung der RWGS Reaktion mittels aus rückgekoppelter Energie durchgeführt, die aus der Verbrennung von Polyurethan-Material Abfall erhalten wird. Unter "rückgekoppelter Energie" versteht der Fachmann Energie, insbesondere Wärmeenergie, die aus einem Verfahrensschritt des erfindungsgemäßen Verfahrens entnommen (gegebenenfalls in einer andere Energieform, z.B. elektrischen Strom, umgewandelt) und in einen anderen Verfahrensschritt des erfindungsgemäßen Verfahrens wieder eingebracht wird.

In einer bevorzugten Variante des neuen Verfahrens erfolgt die Heizung der RWGS Reaktion mittels Verbrennung von Kohlenwasserstoffen aus regenreativer Kohlenwasserstofferzeugung, insbesondere mittels Verbrennung von Biomethan. Unter Biomethan wird hier Methan verstanden, das aus dem durch Vergärung von Biomasse erhaltenen Biogas gewonnen wird. Eine weitere besonders bevorzugte Variante des neuen Verfahrens ist dadurch gekennzeichnet, dass das Polyurethan Material nach seiner Nutzung als Polyurethan-Material Abfall rezykliert wird und der Polyurethan-Material Abfall zu Kohlendioxid verbrannt und das Kohlendioxid als Einsatzmaterial in der Reinigung eingesetzt wird.

Bevorzugt wird der Sauerstoff für die Verbrennung aus einer Wasserelektrolyse erhalten.

Durch bevorzugtem Einsatz von Strom aus regenerativer Energie (bevorzugt aus Windkraft, aus Wasserkraft oder aus Sonnenenergie) wird die CO₂-Emission bei dem Gesamtverfahren weiter herabgesetzt. In einem bevorzugten neuen Verfahren wird der bei der Wasserelektrolyse gebildete Wasserstoff wahlweise bei der optionalen Raffination und/oder bei einer Hydrierung von Nitroverbindungen verwendet, wobei die bei der Hydrierung von Nitroverbindungen erhaltenen Amine in der Isocyanat Herstellung eingesetzt werden können. Der gegebenenfalls in des neuen Verfahrens erhaltene abgetrennte Wasserstoff wird bevorzugt bei der Hydrierung von Nitroverbindungen eingesetzt. Hiermit werden Amine als Vorprodukte des Isocyanats zugänglich.

Der Stoffkreislauf wird in einer besonders bevorzugten Ausführung des erfindungsgemäßen Verfahren weiter dadurch geschlossen, dass das Polyurethan-Material nach seiner Nutzung als Polyurethan-Material Abfall rezykliert wird und der Polyurethan-Material Abfall zu Kohlendioxid verbrannt und das Kohlendioxid als Einsatzmaterial in der Reinigung eingesetzt wird.

Beim Recycling von PU-Material nach dem Ende der Nutzungsdauer werden übliche Trennverfahren zur Abtrennung von Verbundstoffen im Abfall angewandt. So wird das PU-Material automatisiert oder händisch grob abgetrennt, dann mechanisch zerkleinert und ggf. weiter getrennt. Das erhaltene PU-Material dient als Rohstoff Polyurethan-Material Abfall für die Verbrennung oder die Pyrolyse.

Im Falle der Verbrennung wird der Polyurethan-Material Abfall beispielsweise mit reinem Sauerstoff O₂, welches als Nebenprodukt der Wasserelektrolyse, an der Anode entwickelt wird, umgesetzt. Die bei der Verbrennung anfallende Reaktionswärme kann als rückgekoppelte Energie zur Herstellung von Dampf und/oder von elektrischem Strom eingesetzt werden. Besonders kann die Wärme zum Betrieb der Pyrolyse eingesetzt werden und der erzeugte elektrische Strom in der Elektrolyse. Hierdurch wird die Effizienz des neuen Gesamtverfahrens weiter verbessert.

Ebenfalls kann die bei der Verbrennung gewonnene Wärme als rückgekoppelte Energie zur Beheizung der RWGS Reaktion eingesetzt werden, wodurch die energetische Effizienz des neuen Gesamtverfahrens gegenüber dem Stand der Technik weiter verbessert wird.

Das aus der Verbrennung oder aus der Pyrolyse des Polyurethan-Material Abfalles stammende CO₂ fällt in hochkonzentrierter Form an und wird vor der weiteren Nutzung einer Reinigung zugeführt. Hierbei werden die Nebenprodukte der Verbrennung, z.B. Schwefelverbindungen wie SO₂, Stickstoffverbindungen wie NO_{X} sowie restliche Organika als auch Staub und andere entstandene Verbindungen, die aus denen im PU-Material vorhandenen Komponenten entstanden sind, abgetrennt.

Die Verbrennung des Polyurethan-Material Abfalls mit reinem Sauerstoff kann z.B. gemäß dem als Oxyfuel-Verfahren bekannten Verfahren in einer Atmosphäre aus reinem Sauerstoff und CO₂ (rezirkulierendem Rauchgas) erfolgen. Das dabei entstehende Rauchgas ist nicht mit dem in der Luft enthaltenen Stickstoff verdünnt und besteht im Wesentlichen aus CO₂ und Wasserdampf. Der Wasserdampf kann mit wenig Aufwand kondensiert werden, so dass ein hochkonzentrierter CO₂-Strom (Konzentration im Idealfall nahe 100 Prozent) entsteht. Das CO₂ kann dann gereinigt und weiterverarbeitet, ggf. auch verdichtet und gelagert werden.

Weiterhin kann ein Teil der Energie, die bei der Pyrolyse oder bei der Verbrennung des Polyurethan-Material Abfalls gewonnen wird in Dampf oder Elektrizität umgesetzt werden. Mit der gewonnenen Elektrizität kann, wie bereits zuvor erwähnt die Elektrolyse betrieben werden, womit ein noch effizienteres Verfahren mit geringem Verbrauch an elektrischer Energie entsteht.

Die Reinigung des CO₂ aus Verbrennungsgasen kann nach aus dem Stand der Technik grundsätzlich bekannten Verfahren erfolgen. Dies wird im Folgenden beispielhaft beschrieben.

Zuerst erfolgt dabei z.B. eine Reinigung der Verbrennungsgase, deren Hauptbestandteil CO₂ ist. Der Aufbau einer Verbrennungsgasreinigung unterteilt sich in unterschiedliche Stufen. Besondere Aufgabe der Reinigung ist es, ein CO₂ ohne störende Nebenbestandteile für die nachfolgende RWGS Reaktion bereit zu stellen.

In der ersten Stufe wird Staub aus dem Verbrennungsgas entfernt. Dies kann mit Gewebefiltern oder mit einem Elektrofilter erfolgen. Danach können ggf. vorhandene saure Gas, wie Chlorwasserstoff, der sich aus im Abfall vorhandenen Chlor-Verbindungen bildet, entfernt werden. Hierbei werden z.B. Abgaswaschtürme eingesetzt. Das Verbrennungsgas wird hierbei auch abgekühlt und von weiteren Stäuben und ggf. Schwermetallen befreit. Weiterhin wird auch gebildetes Schwefeldioxid-Gas in einem Waschkreislauf abgeschieden und z.B. mit Kalkhydrat zu Gips umgesetzt. Die Entfernung von Stickstoffverbindungen aus den Verbrennungsgasen kann z.B. an katalysatorenthaltenen Zeolithen oder durch Zugabe von Harnstoff oder Ammoniak die Stickoxide wieder zu Stickstoff und Wasser umgewandelt werden. Um eine Entstehung von Ammoniumsalzen zu verhindern, die die Katalysatorporen verstopfen würden, erfolgt der Betrieb der Katalysatoren meist bei einer Temperatur von über 320 °C. Ebenso können die N₂-Verbindungen durch Wäsche mit Salpetersäure oder eine Wäsche mit Katalysatoren entfernt werden.

Die Trocknung und weitere Reinigung des CO₂ kann durch übliche bekannte Verfahren erfolgen. Trocknung z.B. durch eine Behandlung mit konzentrierter Schwefelsäure.

In der letzten Reinigungsstufe werden Aktivkohlefilter eingesetzt, um noch im Verbrennungsgas enthaltene restliche Organika sowie letzte Reste von Metallen durch Aktivkohle zu entfernen. Hierzu kann z.B. staubförmige Aktivkohle in den Verbrennungsgasstrom bzw. Rauchgasstrom dosiert zugeführt und anschließend zusammen mit den angelagerten Schadstoffen auf den Gewebefilter wieder abgeschieden werden. Die verbrauchte Kohle wird ausgeschleust und der energetischen Verwertung zugeführt (grundsätzlich beschrieben in: https://www.ava-augsburg.de/umwelt/rauchgasreinigung/).

Nach den durchgeführten Reinigungsverfahren der Verbrennungsgase steht ein CO₂ zur Verfügung, welches als Rohstoff der RWGS Reaktion eingesetzt werden kann.

Optional kann auch die CO₂ Abtrennung mittels Aminwäsche aus Gasströmen mit geringerer Konzentration an CO₂ erfolgen.

Bei der Pyrolyse ist die Zuführung von zusätzlichem Sauerstoffgas in den Reaktionsraum der Pyrolyse nicht bevorzugt. Die Pyrolyse des gebrauchten Polyurethan Materials kann bevorzugt wie folgt durchgeführt werden:
Die Pyrolyse des Polyurethan Materials erfolgt bei erhöhter Temperatur, gegebenenfalls in Gegenwart von Katalysator, unter Erhalt von gegebenenfalls Kohlendioxid , gegebenenfalls Kohlenmonoxid, gegebenenfalls/ Wasserstoff, einem Gemisch von aliphatischen und aromatischen niedermolekularen Kohlenwasserstoffen und stickstoffhaltigen Kohlenwasserstoffen und einem Rückstand von höher molekularen Kohlenstoffverbindungen,
gegebenenfalls Raffination des erhaltenen Gemisches von niedermolekularen Kohlenwasserstoffen unter Erhalt von einem Gemisch von gasförmigen und flüssigen Kohlenwasserstoffen und einem Gemisch von Kohlendioxid und Kohlenmonoxid, Wasserstoff und weiteren gasförmigen Kohlenwasserstoffverbindungen, und Trennung der erhaltenen Gemische in einer Gastrennung,
Verbrennung des erhaltenen Rückstands und gegebenenfalls von weiterem Polyurethan Materialabfall mit sauerstoffhaltigem Gas, insbesondere mit reinem Sauerstoff, unter Erhalt von Kohlendioxid enthaltendem Gas,
Der wie zuvor beschrieben recycelte und zerkleinerte Polyurethan-Material Abfall kann der Pyrolyse zugeführt werden, wobei die Pyrolyse wahlweise mit oder ohne Katalysator durchgeführt werden kann.

Die bei der Pyrolyse entstehenden Fraktionen sind gasförmig, flüssig und fest, wobei die feste Phase meist vorwiegend aus pyrolytischem Kohlenstoff besteht. Die flüssigen langkettigen KohlenstoffVerbindungen, enthaltend Aromaten wie Toluol, Benzol, Xylol, werden bevorzugt einem Raffinationsprozess zugeführt. Hier können die Verbindungen aufgetrennt oder in Raffinationsprozessen ggf. mit Wasserstoff, vorzugsweise Wasserstoff aus der Wasserelektrolyse weiter umgesetzt werden, so dass im Ergebnis auch Propen und Ethen (als Vorprodukte für Polyole, Polyether) erhalten werden können. Die langkettigen, flüssigen Kohlenwasserstoff-Verbindungen können abgetrennt und weiterverarbeitet werden. Auch die aromatischen Verbindungen wie Benzol oder Anilin oder, falls vorkommend auch Isocyanate, könnten in den entsprechenden Synthesen als Rohstoffe wieder eingesetzt werden.

Weiterhin kann die Pyrolyse wahlweise insbesondere so betrieben werden, dass größere Mengen an Kohlenmonoxid und ggf. Wasserstoff erzeugt werden. Diese Gase können gemeinsam mit den kurzkettigen Kohlenwasserstoff-Verbindungen z.B. in der Raffination abgetrennt oder auch separat abgetrennt werden und dann einer Kohlenmonoxid-Wasserstoff-Trennung zugeführt und verwendet werden.

Die bei der Pyrolyse anfallenden festen Stoffe, bestehen meist aus Kohlenstoff. Diese feste Phase kann mit reinem Sauerstoff aus der Wasserelektrolyse umgesetzt werden. Hierbei entsteht ebenfalls ein hoch konzentrierter Stoffstrom an CO₂, welcher einer Reinigung zugeführt wird.

Eine andere Möglichkeit zur Herstellung von hochreinem CO₂ ist die Absorption des CO₂ in Alkalilauge, zum Beispiel Kalilauge. Hierbei bildet sich Kaliumhydrogencarbonat, welches anschließend wieder zu CO₂ und Kalilauge thermisch zersetzt werden kann. Hierbei kann erzeugte Wärme aus der Pyrolyse oder Verbrennung eingesetzt werden.

Das gereinigte CO₂ wird der RWGS Reaktion zugeführt.

Das Gasgemisch, das der RWGS Reaktion entnommen wird, wird abgekühlt. Dabei wird das Reaktionswasser abgetrennt. Das Reaktionswasser kann als Rohstoff der Wasserelektrolyse wieder zugeführt werden. Nach der Wasser-Abtrennung wird das Gas der CO₂-Abtrennung zugeführt.

Die CO₂ Abtrennung erfolgt z.B. durch eine Aminwäsche, in der das CO₂ entfernt und das Restgas aus CO und H₂ einer H₂/CO Gastrenneinheit zugeführt wird. Das erhaltene CO wird dann der Phosgensynthese zugeführt und hier mit Cl₂ zu Phosgen umgesetzt. Das erzeugte Phosgen wird der Isocyanatherstellung zugeführt. In der Isocyanat-Herstellung wird das Phosgen mit einem Amin zu einem Isocyanat und Chlorwasserstoff umgesetzt.

Der aus der Wasserelektrolyse bzw. der H₂/CO Trennung erhaltene Wasserstoff kann entweder zur Hydrierung der Nitroverbindungen zu den Aminen und damit der Herstellung der Isocyanate zugeführt werden.

Bevorzugt ist daher eine Ausführung des neuen Verfahrens, bei der mindestens Teilströme des Kohlenmonoxids und/oder des Wasserstoffs aus der H₂/CO Trennung einer RWGS Reaktion zugeführt werden.

Das Isocyanat aus der Isocyanatherstellung wird mit Polyetherpolyol oder mit Polyesterpolyol zu Polyurethan-Material in einer entsprechenden Synthese umgesetzt.

Das neue Verfahren kann auch bevorzugt so betrieben werden, dass ein Teil des Polyurethan Material Abfalls anstelle der Pyrolyse direkt der Verbrennung zugeführt wird.

Der bei der Isocyanat-Herstellung anfallende Chlorwasserstoff (HCl) kann einer anderen HCl-Recyclingseinheit wie z.B. einer HCl-Diaphragma oder HCl-Elektrolyse mit Gasdiffusionselektrode oder einer katalytischen Gasphasenoxidation zugeführt werden. Im Falle der HCl-Elektrolyse mit Gasdiffusionselektrode oder einer Gasphasenoxidation kann der benötigte O₂ aus der Wasserelektrolyse bezogen werden.

Dem Fachmann ist die Herstellung von Chlorgas aus elektrochemischer Oxidation nach dem HCl ODC Elektrolyse-Verfahren (geeignete Elektrolysezelle vgl. US,6022,634 A, WO 03/31690 A1), die Herstellung von Chlorgas aus HCl Diaphragmaelektrolyse (vgl. EP 1 103 636 A1), die Herstellung von Chlorgas aus thermokatalytischer Gasphasenoxidation (vgl. WO 2012/025483 A2), sowie die Herstellung von Chlor aus Chloralkali-Elektrolyse (vgl. WO 2009/007366 A2) hinlänglich bekannt. Auf den Inhalt der vorgenannten, im Zusammenhang mit der Herstellung von Chlorgas zitierten Schriften wird ausdrücklich und vollumfänglich Bezug genommen.

Mit den Isocyanaten und den Polyetherpolyolen und gegebenenfalls zusätzlich mit Polyesterpolyolen können dann die im Markt benötigten PU-Materialien hergestellt werden. Die Polyurethane werden in verschiedenen Anwendungen im Markt genutzt. Nach dem Ende ihrer Nutzungszeit werden die Materialien einem Recycling zugeführt und hier die PU-Materialien abgetrennt. Das abgetrennte Material wird dann als Polyurethan-Material Abfall wieder der Verwertung in Form einer Pyrolyse und/oder der Verbrennung zugeführt.

Hierdurch werden keine weiteren fossilen Rohstoffe für die Isocyanat-Herstellung benötigt und es kann in verbessert nachhaltiger Weise Polyurethan-Material hergestellt werden.

Die Erfindung wird nachstehend anhand der Figuren beispielhaft näher erläutert.

Es zeigen:

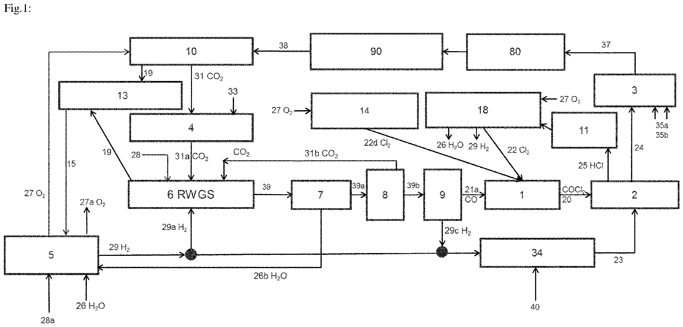

- Fig. 1: Eine schematische Übersicht über das Gesamtverfahren mit RWGS Reaktion, Chlorherstellung, PU Herstellung, Verwendung und Verwertung des Polyurethan-Material Abfalls daraus zu CO₂ für die RWGS Reaktion

In Figur 1 haben die folgenden Bezugszeichen die jeweils rechtsstehende Bedeutung:
- 1: Phosgensynthese
- 2: Isocyanatherstellung
- 3: Polyurethanherstellung
- 4: CO₂ Gasreinigung
- 5: Wasserelektrolyse
- 6: RWGS Reaktion (Reverse Water Gas Shift Reaction)
- 7: Wasser-Abtrennung
- 8: CO₂ Abtrennung
- 9: H₂/CO Trennung
- 10: Verwertung von Polyurethan-Material Abfall (38) durch Pyrolyse (10a) und/oder Verbrennung (10b)
- 10a: Pyrolyse
- 10b: Verbrennung
- 11: HCl Gas Abtrennung / Reinigung
- 12: Elektrochemische Oxidation von HCl mittels HCl-SVK Elektrolyse
- 13: Energie- und Dampferzeugung
- 14: Chlor-Alkali-Elektrolyse zur Herstellung von Cl₂
- 15: elektrischer Strom aus Energie/Dampf (13)
- 16: Cl₂-Herstellung durch thermokatalytische Gasphasenoxidation (Deacon) von HCl
- 17: Cl₂-Herstellung durch Elektrochemische Oxidation von Salzsäure mittels Diaphragma Elektrolyse
- 18: oxidative Umsetzung von HCl zur Chlorgas als (12) und/oder (16) und/oder (17)
- 19: Wärme
- 20: Phosgen
- 21: Kohlenmonoxid im Gasstrom aus der RWGS Reaktion
- 21a: Kohlenmonoxid aus der H₂ CO Trennung
- 22: Chlorgas ausgewählt aus 22a und/oder 22b und/oder 22c
- 22a: Chlorgas aus thermokatalytischer Gasphasenoxidation (16)
- 22b: Chlorgas aus elektrochemischer Oxidation nach dem HCl ODC Verfahren (12)
- 22c: Chlorgas aus HCl Diaphragmaelektrolyse (17)
- 22d: Chlor aus Chloralkali-Elektrolyse (14) (bevozugt mit Sauerstoffverzehrkathode (SVK) unter Sauerstoffzufuhr (27))
- 23: Diamine
- 24: Diisocyanate
- 25: Chlorwasserstoff
- 26: Wasser
- 26b: Wasser
- 27: Sauerstoff
- 27a: Sauerstoff
- 28: Bio-Erdgas u/o regenerative Energie ggf. nur zur Heizung
- 28a: elektrischer Strom aus regenerativer Energie
- 29: Wasserstoff aus der Wasserelektrolyse
- 29a: Wasserstoff aus der Wasserelektrolyse für die RWGS Reaktion
- 29b: Wasserstoff in der RWGS Reaktion nicht umgesetzt
- 29c: Wasserstoff, nicht umgesetzt aus der H₂ CO Trennung
- 31: CO₂ aus 10
- 31a: gereinigtes CO₂
- 31b: nicht umgesetztes CO₂ aus der CO₂ Abtrennung
- 32: Nebenprodukte aus der RWGS Reaktion
- 33: CO₂ aus regenerativen Quellen
- 34: Hydrierung Nitroverbindungen für die Diaminherstellung
- 35a: Polyetherpolyol
- 35b: Polyesterpolyol
- 37: Polyurethan-Material
- 38: Polyurethan-Material enthaltender Abfall (Polyurethan-Material Abfall) für 18
- 39: Produktgasgemisch aus der RWGS Reaktion bestehend aus 21, 26b, CO₂, 29b und 32
- 39a: 39 mit reduziertem Gehalt an 26b
- 39b: 39a mit reduziertem Gehalt an CO₂
- 40: Nitroverbindungen
- 80: Nutzung von 37 im Markt
- 90: Polyurethan basierte Materialien - End-of-Life Recycling

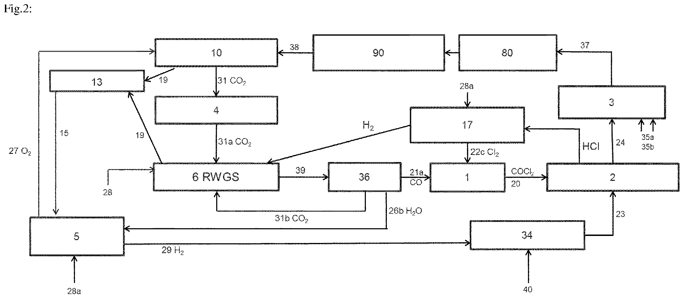

- Fig. 2: Eine schematische Übersicht über das Gesamtverfahren mit RWGS Reaktion, einer Salzsäure-Elektrolyse nach dem Diaphragmaverfahren (HCl-DIA) zur Chlorherstellung, einschließlich der PU Herstellung, Verwendung des Polyurethan-Materials und Verwertung des Polyurethan-Material Abfall daraus zu CO₂ für die RWGS Reaktion.

Die Vergabe der in Fig. 2 verwendeten Bezugszeichen erfolgt wie für Fig.1 definiert.

### Beispiel 1

### Erfindungsgemäße Herstellung von emissionsarmen Toluol-diisocyanat (TDI), CO Herstellung mittels RWGS, deren Beheizung mit Bio-Erdgas erfolgt, HCl Recycling mittels HCl Gasphasenoxidation (Deacon) und H₂ Bereitstellung aus einer Wasserelektrolyse

In einem RWGS Reaktionsraum (6), der bei einer Temperatur von 802°C betrieben wurde, wurden 17,84 t/h CO₂ sowie 0,81 t/h H₂ eingebracht. Aus der RWGS Reaktion wurde das erhaltene Produktgasgemisch (39) bestehend aus CO (21), H₂O (26b), nicht umgesetztes CO₂ sowie nicht umgesetzter H₂ (29a) sowie Nebenprodukten (32), hauptsächlich geringe Mengen an Methan, entnommen und einer Wasser-Abtrennung (7) zugeführt, bei der 7,3 t/h Wasser erhalten wurden. Dieses Wasser (26b) wird der Wasserelektrolyse (5) wieder zugeführt. Aus der Wasserelektrolyse (5) wurden insgesamt 3,24 t/h Wasserstoff entnommen, so dass zusätzlich 21,86 t/h Wasser zugesetzt wurden. Das verbleibende Gasgemisch (39a) aus der RWGS wurde einer CO₂ Abtrennung (8) zugeführt. Die CO₂ Abtrennung erfolgte mittels Aminwäsche, wobei das abgetrennte CO₂ (31b) wieder der RWGS Reaktion zugeführt wurde. Die Energie zur CO₂ Abtrennung aus dem gebildeten CO₂-Aminkomplex wurde aus der Wasser-Abtrennung (7) der RWGS Gase (39) bezogen. Das von CO₂ befreite Gas (39b) wurde der H₂-CO-Trennung (9) zugeführt. Für die H₂-CO Trennung wurde eine sogenannte Cold-box eingesetzt, bei der das H₂-CO Gasgemisch abgekühlt und Wasserstoff und CO getrennt wurden. Der abgetrennte Wasserstoff (29c) wurde der RWGS (6) wieder zugeführt. Aus der H₂-CO Trennung (9) wurden 11,35 t/h CO einer Phosgensynthese (1) zugeführt. Hierbei reagierte das CO mit 29,79 t/h Chlor, welches aus einer HCl-Gasphasenoxidation (16) entnommen wurde. Aus der Phosgensysnthese (1) wurden 40,15 t/h Phosgen entnommen und in einer Isocyanat-Herstellung (2) mit 24,73 t/h Toluol-diamin 23 zu 35,27 t/h Toluol-diisocyanat (24) umgesetzt. Das dabei anfallende HCl-Gas (25) mit einer Menge von 29,59 t/h wurde nach Reinigung über eine Tieftemperaturdestillation einer HCl-Gasphasenoxidation (16) zugeführt. In der HCl-Gasphasenoxidation (16) wurde das HCl Gas bei ca. 300°C an einem Rutheniumoxid-basierten Katalysator mit Sauerstoff (27) zu Chlor und H₂O umgesetzt. Der benötigte Sauerstoff (27) wurde aus der Wasserelektrolyse (5) entnommen. Das erhaltene Toluol-diisocyanat (24) wurde in üblicher Weise mit Polyetherpolyolen (35a) oder Polyesterpolyolen (35b) zu Polyurethan-Material (37) umgesetzt.

Nach Nutzung des Polyurethan-Materials in diversen Anwendungen im Markt (80), konnte dieses gesammelt, und recycelt (90) werden, um den daraus erhaltenen Polyurethan-Material Abfall (38) einer Verbrennung (10b) zuzuführen. Die Verbrennung wurde dabei mit Sauerstoff (27) aus der Wasserelektrolyse (5) umgesetzt, so dass ein hochkonzentrierter CO₂ Abgasstrom (31) entstand. Dieser CO₂ Strom (31) wurde einer CO₂ Reinigung (4) zugeführt und dabei das aus der Verbrennung stammende Wasser entfernt und Stickstoffoxide sowie Schwefeloxide abgetrennt. Hiernach wurden 17,84 t/h CO₂ der RWGS (6) zugeführt.

Der Wasserstoff (29) wurde in einer Wasserelektrolyse mit einer Leistung von 45 MW erzeugt, wobei regenerative Energie eingesetzt wurde. Die Wasserelektrolyse (5) war eine alkalische Wasserelektrolyse, die mit einer Stromdichte von 8 kA/m² und einer Zellspannung von 2 V je Elektrolyseelement betrieben wurde. Dabei wurden 45 MW und 21,86 t/h Wasser sowie 7,3 t/h Wasser aus H₂O Abtrennung (7) zugeführt. Der Wasserelektrolyse wurden 3,24 t/h H₂ entnommen.

Die RWGS Reaktion wurde bei 802°C betrieben, die Temperatur wurde durch Verbrennung mit Bio-Erdgas erzeugt.

Durch das erfindungsgemäße Verfahren wurden 22% des Kohlenstoffs, der im TDI vorhanden ist, aus einer nichtfossilen Kohlenstoffquelle ersetzt. Durch Einsatz von regenerativer Energie bei der Wasserelektrolyse wurde der CO₂-Footprint des aus CO und Cl₂ hergestellten Phosgens weiterhin erniedrigt.

### Beispiel 2

### Erfindungsgemäße Herstellung von emissionsarmen Toluol-diisocyanat (TDI), CO Herstellung mittels RWGS, deren Beheizung mit Bio-Erdgas erfolgt, HCl Recycling mittels HCl-Diaphragmaelektrolyse und H₂ Bereitstellung für die Hydrierung von Dinitrotoluol aus einer Wasserelektrolyse

In einem RWGS Reaktionsraum (6), der bei einer Temperatur von 802°C betrieben wurde, wurden 17,84 t/h CO₂ sowie 0,81 t/h H₂ eingebracht. Der Wasserstoff stammte dabei aus dem HCl-Recycling durch eine HCl-Diaphragmaelektrolyse. Aus der RWGS wurde das erhaltene Produktgasgemisch (39) bestehend aus CO (21), H₂O (26b), nicht umgesetztes CO₂ sowie nicht umgesetzter H₂ (29a) sowie Nebenprodukten (32), hauptsächlich geringe Mengen an Methan, entnommen und einer Wasser-Abtrennung (7)zugeführt, bei der 7,3 t/h Wasser erhalten wurden. Dieses Wasser (26b) wurde der Wasserelektrolyse (5) wieder zugeführt. Aus der Wasserelektrolyse wurden insgesamt 2,43 t/h Wasserstoff entnommen, so dass zusätzlich 14,56 t/h Wasser zugesetzt wurden. Das verbleibende Gasgemisch (39a) aus der RWGS wurde einer CO₂ Abtrennung (8) zugeführt. Die CO₂ Abtrennung erfolgte mittels Aminwäsche, wobei das abgetrennte CO₂ (31b) wieder der RWGS zugeführt wurde. Die Energie zur CO₂ Abtrennung aus dem gebildeten CO₂-Aminkomplex wurde aus der Wasser-Abtrennung (7) der RWGS Gase (39) bezogen. Das von CO₂ befreite Gas (39b) wurde der H₂-CO-Trennung (9) zugeführt. Für die H₂-CO Trennung wurde eine sogenannte Cold-box eingesetzt, bei der das H₂-CO Gasgemisch abgekühlt und Wasserstoff und CO getrennt wurden. Der abgetrennte Wasserstoff (29c) wurde der RWGS (6) wieder zugeführt. Aus der H₂-CO Trennung (9) wurden 11,35 t/h CO einer Phosgensynthese (1) zugeführt. Hierbei reagierte das CO mit 29,79 t/h Chlor, welches aus einer HCl-Diaphragmaelektrolyse (17) entnommen wurde. Aus der Phosgensysnthese (1) wurden 40,15 t/h Phosgen entnommen und in einer Isocyanat-Herstellung (2) mit 24,73 t/h Toluol-diamin 23 zu 35,27 t/h Toluol-diisocyanat (24) umgesetzt. Das dabei anfallende HCl-Gas (25) mit einer Menge von 29,59 t/h wurde nach Reinigung über eine Tieftemperaturdestillation, Absorption in Wasser unter Bildung einer 35%igen Salzsäure, Reinigung der Salzsäure mittels Aktiv-Kohle einer HCl-Diaphragmaelektrolyse (17) zugeführt. Aus der HCl-Diaphragmaelektrolyse wurden Chlor und Wasserstoff entnommen. Der Wasserstoff wurde gereinigt und der RWGS zugeführt. Das erhaltene Toluol-diisocyanat (24) wurde in üblicher Weise mit Polyetherpolyolen (35a) oder Polyesterpolyolen (35b) zu Polyurethan-Material (37) umgesetzt.

Nach Nutzung des Polyurethan-Materials in diversen Anwendungen im Markt (80), kann diese gesammelt, und recycelt (90) werden, um den daraus erhaltenen Polyurethan-Material Abfall (38) einer Verbrennung (10b) zuzuführen. Die Verbrennung wurde dabei mit Sauerstoff (27) aus der Wasserelektrolyse (5) umgesetzt, so dass ein hochkonzentrierte CO₂ Abgasstrom (31) entsteht. Dieser CO₂ Strom (31) wurde einer CO₂ Reinigung (4) zugeführt und dabei das aus der Verbrennung stammende Wasser entfernt und Stickstoffoxide sowie Schwefeloxide abgetrennt. Hiernach wurden 17,84 t/h CO₂ der RWGS (6) zugeführt.

Der Wasserstoff (29) wurde in einer Wasserelektrolyse mit einer Leistung von 45 MW erzeugt, wobei regenerative Energie eingesetzt wurde. Die Wasserelektrolyse (5) war eine alkalische Wasserelektrolyse, die mit einer Stromdichte von 8 kA/m² und einer Zellspannung von 2 V je Elektrolyseelement betrieben wurde. Dabei wurden 45 MW und 21,86 t/h Wasser sowie 7,3 t/h Wasser aus H₂O Abtrennung (7) zugeführt. Der Wasserelektrolyse wurden 3,24 t/h H₂ entnommen.

Die RWGS wurde bei 802°C betrieben, die Temperatur wurde durch Verbrennung mit Bio-Erdgas erzeugt.

Durch das erfindungsgemäße Verfahren wurden 22% des Kohlenstoffs, der im TDI vorhanden ist, aus einer nichtfossilen Kohlenstoffquelle ersetzt. Durch Einsatz von regenerativer Energie bei der Wasserelektrolyse wurde der CO₂-Footprint des aus CO und Cl₂ hergestellten Phosgens weiterhin erniedrigt.

## Patentansprüche

1. Verfahren zur Herstellung von Isocyanaten und Polyurethanen durch
Synthese (1) von Phosgen (20) aus Kohlenmonoxid (21a) und Chlor (22),
Umsetzung (2) von Phosgen (20) mit Diaminen (23) zu Diisocyanaten (24) und Chlorwasserstoff (25),
Umsetzung (3) der Diisocyanate (24) mit Polyetherpolyol (35a) und/oder Polyesterpolyol (35b) zu Polyurethanen (37),
Bereitstellung eines CO₂ Gasstroms (31),
Reinigung (4) des CO₂ Gasstroms (31) von Nebenbestandteilen, insbesondere von Stickoxiden, Schwefelverbindungen, Staub, Wasser, Sauerstoff und HCl wahlweise mittels Adsorption, Gaswäsche oder katalytischer Behandlung unter Erhalt eines gereinigten Kohlendioxids (31a),
Elektrolyse (5) von Wasser (26) zu Wasserstoff (29) und Sauerstoff (27),
Bereitstellung des Wasserstoffstroms (29a) und Einspeisung zusammen mit dem gereinigten CO₂ Gasstrom (31a) in eine RWGS Reaktionszone und Umsetzung (6) der Edukte nach dem Prinzip der RWGS zu einem Produktgasgemisch (39) aus Wasserdampf (26b), CO (21) und gegebenenfalls Nebenprodukten (32), insbesondere niedere Kohlenwasserstoffe, insbesondere bevorzugt Methan,
Abtrennung (7) des Wassers des Wasserdampfes (26b) aus dem Produktgasgemisch (39) und Rückführung des Wassers in die Wasserelektrolyse (5),
Abtrennung (8) von nicht umgesetztem Kohlendioxid (31b) aus dem aus der Abtrennung (7) erhaltenen Gasgemischs (39a) der RWGS Reaktion (6), insbesondere mittels Aminwäsche, und Rückführung des nicht umgesetzten Kohlendioxids (31b) in die RWGS Reaktion (6),
Abtrennung (9) des in der RWGS Reaktion (6) nicht umgesetzten Wasserstoffs (29a) aus dem nach der Abtrennung (8) erhaltenen Gasgemisch (39b) von Kohlenmonoxid (21a) und Wasserstoff (29c), insbesondere unter Verwendung einer Coldbox, und wahlweise Rückführung des Wasserstoffs (29c) in die RWGS Reaktion (6) oder Einspeisung des Wasserstoffs (29c) in die Hydrierung (34) von Di-Nitroverbindungen zur Herstellung von Diaminen (23) als Rohstoff für das Diisocyanat (24),
Einspeisung des verbliebenen Kohlenmonoxids (21a) aus der Abtrennung (9) in die Phosgensynthese (1),
Einspeisung des Wasserstoffs (29) aus der Wasserelektrolyse (5), gegebenenfalls zusammen mit nicht umgesetztem Wasserstoff (29 c) aus der RWGS Reaktion (6) in die Hydrierung von Nitroverbindungen (34) zur Herstellung (2) von Diaminen (23),
Abtrennung und Reinigung (11) des in der Isocyanat-Herstellung (2) gebildeten Chlorwasserstoffs (25) und anschließende oxidative Umsetzung (18) des Chlorwasserstoffs (25) als Umsetzung in einer thermokatalytischen Gasphasenoxidation (16) mit Sauerstoff (27) zu Chlor (22a) und Wasser (26), und/oder als elektrochemische Oxidation (12) des Chlorwasserstoffs (25) zu Chlor (22b) und/oder als elektrochemische Oxidation (17) des Chlorwasserstoffs (25) zu Chlor (22c) und Wasserstoff (29) mittels HCl Diaphragmaelektrolyse (17),
Zuführung des zuvor gebildeten Chlors (22a, 22b, 22c) in die Phosgensynthese (1) gegebenenfalls unter Zuspeisung von frischem Chlor (22d) aus einer Chloralkali-Elektrolyse (14).

2. Verfahren gemäß Anspruch 1, **dadurch gekennzeichnet, dass** für die RWGS-Synthese (6) Kohlendioxid (31) verwendet wird, das aus der Verwertung (10) von Polyurethan-Material Abfall (38) durch Verbrennung (10b) und/oder durch Pyrolyse (10a) bereitgestellt wird.

3. Verfahren gemäß Anspruch 2, **dadurch gekennzeichnet, dass** die Verbrennung (10b) unter Verwendung von Gas mit einem Sauerstoffgas-Gehalt (O₂) von mindestens 30 Vol.% durchgeführt wird, insbesondere unter Einsatz von Sauerstoffgas (27a), das aus der Wasserelektrolyse (5) erhalten wird.

4. Verfahren gemäß Anspruch 2, **dadurch gekennzeichnet, dass** das Gas einen Sauerstoffgas-Gehalt (O₂) von mindestens 50 Vol.%, bevorzugt von mindestens 95 Vol.%, besonders bevozugt von mindestens 99 Vol.%, ganz besonders bevorzugt von mindestens 99,5 Vol.%, aufweist.

5. Verfahren gemäß einem der Ansprüche 1 bis 4, **dadurch gekennzeichnet, dass** die Wasserelektrolyse (5) und/oder die elektrochemische Oxidation (12, 17) unter Verwendung von aus regenerativer Energie erzeugtem elektrischem Strom durchgeführt wird, insbesondere aus regenerativer Energie in Form von Windkraft, Sonnenenergie oder Wasserkraft.

6. Verfahren gemäß einem der Ansprüche 1 bis 5, **dadurch gekennzeichnet, dass** die Wasserelektrolyse (5) und/oder die elektrochemische Oxidation (12, 17) unter Verwendung von elektrischem Strom aus rückgekoppelter Energie durchgeführt wird, die bei der Verbrennung von Polyurethan-Material Abfall und/oder der Durchführung der RWGS Reaktion erhalten wird.

7. Verfahren gemäß einem der Ansprüche 1 bis 6, **dadurch gekennzeichnet, dass** die RWGS Reaktion (6) mittels aus regenerativer Energie erzeugten elektrischen Stroms (28) durchgeführt wird, insbesondere elektrischem Strom wahlweise erhalten durch Nutzung von Windkraft, Sonnenenergie oder Wasserkraft.

8. Verfahren gemäß einem der Ansprüche 1 bis 7, **dadurch gekennzeichnet, dass** die Heizung der RWGS Reaktion (6) mittels aus rückgekoppelter Energie durchgeführt wird, die aus der Verbrennung von Polyurethan-Material Abfall (38) erhalten wird.

9. Verfahren gemäß einem der Ansprüche 1 bis 8, **dadurch gekennzeichnet, dass** die Heizung der RWGS Reaktion (6) mittels Verbrennung von Kohlenwasserstoffen aus regenerativer Herkunft, insbesondere mittels Verbrennung von Biomethan, erfolgt.

10. Verfahren gemäß Anspruch 9, **dadurch gekennzeichnet, dass** das Polyurethan Material (37) nach seiner Nutzung als Polyurethan-Material Abfalls (38) rezykliert wird, und der Polyurethan-Material Abfall (38) zu Kohlendioxid (31) verbrannt und das Kohlendioxid (31) als Einsatzmaterial in der Reinigung (4) eingesetzt wird.
